# EUROPEAN PATENT APPLICATION

(11) **EP 3 824 896 A1**
(43) Date of publication of application: **26.05.2021**
(21) Application number: 19836963.9
(22) Date of filing: 19.07.2019
(51) Int. Cl.: A61K 36/534, A61K 8/73, A61K 8/92, A61K 31/718, A61K 36/232, A61K 36/32, A61K 36/38, A61K 36/483, A61K 36/53, A61K 36/54, A61K 36/61, A61K 36/752, A61K 36/85, A61K 36/899, A61K 36/9062, A61K 36/9064

(54) **VIRUS INACTIVATING AGENT**

(30) Priority: 20.07.2018 JP 2018136872
(71) Applicant: Shiseido Company, Ltd., Chuo-ku Tokyo 104-0061 (JP)
(72) Inventor: ARINO, Shoko, Tokyo 104-0061 (JP); SHIRAKAMI, Hirohito, Tokyo 104-0061 (JP); HIRUMA, Yukiko, Tokyo 104-0061 (JP)
(74) Representative: Ter Meer Steinmeister & Partner
(86) International application number: PCT/JP2019/028379
(87) International publication number: WO 2020/017619

(57) **Abstract**

The purpose of the present invention is to provide: a virus inactivating agent that exhibits an effect at an amount that does not cause skin irritation; and an external skin preparation composition containing said virus inactivating agent. The present invention relates to: a virus inactivating agent containing, as an effective component thereof, at least one selected from mint oil, eucalyptus oil, rosemary oil, sage oil, tea tree oil, shell ginger oil, peppermint oil, lemongrass oil, cajeputi oil, niaouli cineole oil, lime oil, lemon oil, lemon verbena oil, St. John's wort oil, ravintsara oil, rosewood oil, Melissa/lemon balm oil, myrrh oil, mandarin oil, vetiver oil, frankincense oil, citronella oil, cardamom oil, Angelica oil, and cationized starch; and an external skin preparation composition containing the same.

## Description

### Technical Field

The present invention relates to a virus inactivating agent comprising, as active ingredient, one or two or more selected from specific essential oils such as mint oil, eucalyptus oil, rosemary oil, sage oil, tea tree oil, getto oil, peppermint oil, lemongrass oil, cajeput oil, niaouli cineole oil, lime oil, lemon oil, lemon verbena oil, St. John's wort oil, ravintsara oil, rosewood oil, melissa/lemon balm oil, myrrh oil, mandarin oil, vetiver oil, frankincense oil, citronella oil, cardamon oil, and angelica oil, and cationic starch, and a skin external-preparation composition comprising the virus inactivating agent.

### Background Art

Some plant extracts, in particular, some essential oils obtained by extracting aromatic substances contained in plant extracts are known to exhibit an inactivation effect on various viruses including influenza virus. "Essential oil" is one of natural fragrances in cosmetics, and in a narrow sense, it refers to the oil obtained by subjecting a plant or a dried material thereof to steam distillation. As a method for collecting natural fragrances, extraction, expression, and the like are known, and in some cases, the fragrance obtained by expression is referred to as essential oil (Non-Patent Document 1).

For example, Patent Document 1 describes that one or two or more plant extracts selected from raspberry (Rubus idaeus), strawberry (Fragaria vesca), blackberry (Rubus fruticosus), common fig (Ficus carica), lambsquarters (Chenopodium album), agrimony (Agrimonia eupatoria), eucalyptus (Eucalyptus globulus), peach (Prunus persica), apple (Malus pumila), sweet violet (Viola odorata), kuromoji (Lindera umbellata), guarana (Paullinia cupana), watafujiutsugi (Buddleia officinalis), asiatic dayflower (Commelina communis), nazuna (Capsella bursapastoris), isodon herb (Rabdosia japonica), wild strawberry (Fragaria vesca), horehound (Marrubium vulgare), marsh mallow (Althaea officinalis), Asiatic plantain (Plantago asiatica), lemon verbena (Aloysia triphylla), yarrow (Achillea millefolium), icelandic moss (Cetraria islandica), amachazuru (Hydrangea serrata), and Japanese butterbur (Petasites j aponicus) exhibit an influenza virus inactivation effect. However, the plant extract used in Patent Document 1 is obtained by extraction using water/ethanol (or an organic solvent), and it is intended to be added to food products and administered orally.

Patent Document 2 describes that the essential oil component obtained by the steam distillation of dokudami (Houttuynia cordata Thunb) exhibits inactivation effects on influenza virus, Herpes simplex virus type-1 (HSV-1), AIDS virus (HIV), and the like, and Patent Document 3 describes that the essential oil extract from patchouli (Pogostemon cablin Benth.) exhibits an influenza virus inactivation effect. However, Patent Document 2 specifies, as the active ingredients, non-terpene compounds such as n-decylaldehyde, n-dodecylaldehyde, and methyl n-nonylketone contained in essential oil components, and the active ingredient in Patent Document 3 is said to be patchouli alcohol (patchoulol) that can be obtained by extracting patchouli with alcohol or n-hexane, followed by fractionation.

Patent Document 4 discloses that a momi fir leaf extract (momi fir essential oil) is useful as an anti-influenza virus agent, and it is said to be preferable to contain 30% by mass or more of bornyl acetate in the essential oil and also preferable to contain 90% by mass or more of the total amount of bornyl acetate in the amount described above and camphene, pinene, and limonene, based on the total amount of the momi fir essential oil. Patent Document 5 reports that terpene-based derivatives such as pinenes, phenols such as eugenol, and sandalwood oil exhibit inactivation effects on enveloped pathogenic viruses such as measles virus.

However, regarding essential oils, it is not practical to make them contain an effective amount to inactivate the virus in terms of safety (skin irritation), and thus, the development of a virus inactivating agent containing safe natural components that exert their effects in a smaller amount has been strongly desired.

On the other hand, it is reported that a quaternary ammonium cation containing silicon such as dimethyloctadecyl[3-(triethoxysilyl)propyl]ammonium chloride (EtAC) is safe as an oral cavity cleaning agent such as a denture cleanser and also has the ability to inactivate viruses such as influenza virus and Norovirus although it is not a natural component (Patent Document 6). However, Patent Document 6 suggests that only a slight difference in the structure of the quaternary ammonium cation may cause problems in the anti-virus effect and stability (paragraphs 0008 and 0009).

### Citation List

### Non-Patent Document

Non-Patent Document 1: "New Cosmetic Science", second edition, edited by Takeo Mitsui, Nanzando Co., Ltd., 2001, p. 119

### Patent Document

Patent Document 1: Japanese Patent Laid-Open No. 2004-59463
Patent Document 2: Japanese Patent Laid-Open No. 7-118160
Patent Document 3: Japanese Patent Laid-Open No. 2011-79800
Patent Document 4: Japanese Patent Laid-Open No. 2011-84503
Patent Document 5: Japanese Patent Laid-Open No. 5-306217
Patent Document 6: Japanese Patent Laid-Open No. 2011-98976

### Summary of Invention

### Technical Problem

In the technological state as described above, the present invention has been accomplished in view of the current situation in which not only bacteria and viruses are present, but also irritating substances which may cause adverse effects on the skin, such as air pollutants like pollen and PM 2.5 are increasing, and it is an object of the present invention to provide a virus inactivating agent which exerts its effect in an amount to the extent that causes no skin irritation, and a skin external-preparation composition comprising the virus inactivating agent.

### Solution to Problem

As a result of intensive studies to solve the above problems, the present inventors have found that inclusion of one or two or more essential oils extracted from specific plants and/or cationic starch as active ingredient provides a safe and excellent virus inactivation effect without causing skin irritation, and thereby completed the present invention.

More specifically, the present invention comprises the following.
[1] A virus inactivating agent comprising, as active ingredient, one or two or more selected from mint oil, eucalyptus oil, rosemary oil, sage oil, tea tree oil, getto oil, peppermint oil, lemongrass oil, cajeput oil, niaouli cineole oil, lime oil, lemon oil, lemon verbena oil, St. John's wort oil, ravintsara oil, rosewood oil, melissa/lemon balm oil, myrrh oil, mandarin oil, vetiver oil, frankincense oil, citronella oil, cardamon oil, angelica oil, and cationic starch.
[2] The virus inactivating agent according to the above [1] comprising, as active ingredient, one or two or more selected from the group consisting of mint oil, eucalyptus oil, rosemary oil, and sage oil.
[3] The virus inactivating agent according to the above [2] comprising, as active ingredient, any three selected from the group consisting of mint oil, eucalyptus oil, rosemary oil, and sage oil.
[4] The virus inactivating agent according to the above [2] comprising, as active ingredient, mint oil, eucalyptus oil, rosemary oil, and sage oil.
[5] The virus inactivating agent according to any one of the above [1] to [4], wherein a total content of limonene is 0.006% by mass or more.
[6] The virus inactivating agent according to the above [1] comprises the cationic starch as an active ingredient.
[7] The virus inactivating agent according to the above [1] or [6], wherein the cationic starch is a cationic polymer represented by the following formula (I):
   [Formula 1] wherein X- represents an anion derived from an inorganic acid or an organic acid; a is 0.6 to 0.9 and b is 0.4 to 0.1, provided that a and b satisfy a + b = 1; and an average molecular weight is 30,000 to 1,000,000.
[8] The virus inactivating agent according to any one of the above [1], [6] and [7], wherein an average molecular weight of the cationic starch is 100,000 to 500,000.
[9] The virus inactivating agent according to any one of the above [1] and [6] to [8], wherein the cationic starch is starch hydroxypropyltrimonium chloride.
[10] The virus inactivating agent according to any one of the above [1] and [6] to [9], wherein a content of the cationic starch is 0.1% by mass or more in terms of pure starch hydroxypropyltrimonium chloride.
[11] The virus inactivating agent according to any one of the above [1] to [10], wherein the virus is an enveloped single-stranded RNA virus.
[12] The virus inactivating agent according to the above [11], wherein the enveloped single-stranded RNA virus is a virus belonging to family Orthomyxoviridae.
[13] The virus inactivating agent according to the above [12], wherein the virus belonging to family Orthomyxoviridae is influenza virus.
[14] A skin external-preparation composition comprising the virus inactivating agent according to any one of the above [1] to [13].
[15] The skin external-preparation composition according to the above [14], further comprising an alcohol.
[16] The skin external-preparation composition according to the above [15], wherein a content of the alcohol is 50% by mass or less.

### Advantageous Effects of Invention

According to one aspect of the virus inactivating agent of the present invention, the virus inactivating agent exhibits an excellent virus inactivation effect without causing skin irritation, by preferably combining a plurality of essential oils having a low limonene content to increase the total amount of limonene to a predetermined amount or more. Also, the virus inactivating agent of the present invention exhibits an excellent virus inactivation effect by including cationic starch which is a naturally-derived component. Therefore, a skin external-preparation composition comprising the virus inactivating agent of the present invention has the advantage of having virus inactivation action safely and easily.

### Description of Embodiments

Hereinafter, the present invention will be described further in detail.

One aspect is that the virus inactivating agent of the present invention comprises, as active ingredient, one or two or more plant extracts, wherein the plant extract derives from a plant, selected from the group consisting of mint (family Lamiaceae, genus Mentha), eucalyptus (family Myrtaceae, genus Eucalyptus), rosemary (family Lamiaceae, genus Rosmarinus), sage (family Lamiaceae, genus Salvia), tea tree (family Myrtaceae, genus Melaleuca), getto (family Zingiberaceae, genus Alpinia), peppermint (family Lamiaceae, genus Mentha), lemongrass (family Poaceae, genus Cymbopogon), cajeput (family Myrtaceae, genus Melaleuca), niaouli cineole (family Myrtaceae, genus Melaleuca), lime (family Rutaceae, genus Citrus), lemon (family Rutaceae, genus Citrus), lemon verbena (family Verbenaceae, genus Aloysia), St. John's wort (family Guttiferae, genus Hypericum), ravintsara (family Lauraceae, genus Cinnamomum), rosewood (family Fabaceae, genus Dalbergia), melissa/lemonbalm (family Lamiaceae, genus Melissa), myrrh (family Burseraceae, genus Commiphora), mandarin (family Rutaceae, genus Citrus), vetiver (family Poaceae, genus Chrysopogon), frankincense (family Burseraceae, genus Boswellia), citronella (family Poaceae, genus Cymbopogon), cardamon (family Zingiberaceae, genus Elettaria), and angelica (family Apiaceae, genus Angelica).

As the plant extract in the present invention, an "essential oil" extracted as an aromatic substance contained in the above-mentioned plants is preferable.

The essential oil in a narrow sense obtained by steam distillation from the above plants or dried materials thereof is preferably used as the "essential oil" in the present invention, but is not limited thereto. For example, oils extracted from the plants by using other methods such as extraction or expression are also included in the "essential oil" of the present invention as long as they contain essential oil components (such as aromatic substances).

As other methods for extracting essential oils from plants, for example, solvent extraction (such as alcohol extraction, organic solvent extraction), oil and fat adsorption extraction (hot enfleurage or cold enfleurage), and supercritical fluid extraction are known. When the steam distillation cannot be applied because of a low essential oil content in the plant and the like, the solvent extraction is often used. Examples of the solvent used for extraction include, but are not limited to, alcohols such as ethanol, methanol, propanol, isopropanol, and butanol, and organic solvents including relatively high polarity solvents such as acetone and low polarity solvents such as hexane. Regarding the details of these methods for extracting essential oils, reference can be made to publications such as "Patent Office Report: Collection of Well-Known Prior Arts (flavors and fragrances), Part 1, General fragrance" (Jan. 29, 1999, published by the Japan Patent Office, pp. 4 - 21 (2.1.1 plant fragrances)).

The "essential oil" in the present invention may be those in which the oil obtained by the above method is further purified and concentrated by using various purification procedures such as hydrophobic or adsorptive chromatography using a support such as porous beads, silica gel, or alumina.

As used herein, each "essential oil" obtained from each plant listed in paragraph 0015 refers to mint oil, eucalyptus oil, rosemary oil, sage oil, tea tree oil, getto oil, peppermint oil, lemongrass oil, cajeput oil, niaouli cineole oil, lime oil, lemon oil, lemon verbena oil, St. John's wort oil, ravintsara oil, rosewood oil, melissa/lemon balm oil, myrrh oil, mandarin oil, vetiver oil, frankincense oil, citronella oil, cardamon oil, and angelica oil.

The virus inactivating agent of the present invention comprises, as active ingredient, one or two or more essential oils selected from the group consisting of mint oil, eucalyptus oil, rosemary oil, sage oil, tea tree oil, getto oil, peppermint oil, lemongrass oil, cajeput oil, niaouli cineole oil, lime oil, lemon oil, lemon verbena oil, St. John's wort oil, ravintsara oil, rosewood oil, melissa/lemon balm oil, myrrh oil, mandarin oil, vetiver oil, frankincense oil, citronella oil, cardamon oil, and angelica oil. It is preferable to comprise preferably two, more preferably three, and further preferably four or more essential oils.

Among the above-mentioned essential oils, it is preferable to select the essential oil in which the content of limonene is 10 g or less, preferably 8 g or less, and more preferably 6 g or less per 100 g of the essential oil. In particular, it is preferable to use at least one selected from the group consisting of mint oil, eucalyptus oil, rosemary oil, and sage oil. A preferred aspect of the virus inactivating agent of the present invention comprises, as active ingredient, two, preferably three, and more preferably four selected from the group consisting of mint oil, eucalyptus oil, rosemary oil, and sage oil.

The virus inactivating agent of the present invention preferably contain essential oils in combination so that the content of limonene contained in the essential oils is 0.006% by mass or more based on the total amount of the virus inactivating agent. If the content of limonene is less than 0.006% by mass, a sufficient virus inactivation effect cannot be obtained.

Limonene is a monocyclic monoterpene hydrocarbon mainly contained in fruit peels of citrus fruits. Limonene is a main component of the essential oils obtained from citrus fruits such as oranges and lemons, and it is known that the limonene contained in orange peel oil and lemon oil is D-form and the limonene contained in mint oil and the like is L-form. The limonene used in the present invention may be D-form, L-form, or a mixture of D-form and L-form (racemate, etc.) and is not particularly limited. More specifically, the virus inactivating agent of the present invention includes an aspect of containing 0.006% by mass or more of D-limonene, an aspect of containing 0.006% by mass or more of L-limonene, and an aspect of containing D-limonene and L-limonene in a total amount of 0.006% by mass or more.

The upper limit of the content of limonene is not particularly limited, but is typically 0.05% by mass or less, preferably 0.04% by mass or less, 0.03% by mass or less, or 0.02% by mass or less. Too high a content of limonene may cause skin irritation.

The amount of limonene based on the total amount of essential oil to be included is less than 5 g, preferably less than 4.5 g per 100 g of the essential oil.

Another aspect of the virus inactivating agent of the present invention comprises cationic starch as an active ingredient.

The cationic starch is a cationic polymer obtained by introducing a cationic group such as quaternary ammonium salt into a starch having a structure in which a plurality of glucose is linked via α-1,4-glucoside bonds as the basic skeleton.

Preferred examples of the cationic starch used in the present invention include the cationic polymer represented by the following formula (I):

### [Formula 2]

Each symbol in the above formula (I) represents the following meanings.

X- indicates an anion derived from an inorganic acid or an organic acid. Examples of the inorganic acid include hydrochloric acid, sulfuric acid, and nitric acid, and examples of the organic acid include carboxylic acids such as acetic acid. As for the anion X- in the above formula (I), halide ions, in particular, C1- are preferred.

a and b represent each number of monomers and the ratio of each monomer in the polymer. In representing each number of monomers, the average molecular weight of the cationic starch represented by Formula (I) (the weight average molecular weight: Mw) is preferably 30,000 to 1,000,000 and more preferably 100,000 to 500,000, and therefore, a and b take values such that the average molecular weight can be within the above-mentioned range. In representing the ratio of each monomer, for example, a is 0.6 to 0.9, preferably 0.7 to 0.8, and more preferably about 0.75, and b is 0.4 to 0.1, preferably 0.3 to 0.2, and more preferably about 0.25, provided that a + b = 1.

The cationic starch represented by Formula (I) is "starch hydroxypropyltrimonium chloride" in a cosmetic labeling name. Examples of the products commercially available under this labeling name include "Sensomer CI-50" (manufactured by NALCO Performance Products), "DOCCTARCH CP"
(manufactured by DOC Japan), "amylomer 25L" (manufactured by Graefe Chemie), "amylomer 50M" (manufactured by Graefe Chemie), "FARMAL MS5940" (manufactured by Corn Products International), and "EXCEL FM-004" (manufactured by NIPPON STARCH CHEMICAL CO., LTD.), and these commercial products can be used. Among these, "Sensomer CI-50" (average molecular weight = 200,000; a:b = 0.75:0.25) can be preferably used.

Although the cationic starch is mainly used as a hair conditioning agent as a cationic polymer along with cationic cellulose, polyquaternium, and the like, the fact that cationic starch has the virus inactivation effect is a surprising finding that has been found out for the first time by the present invention.

In the virus inactivating agent of the present invention, the content of the cationic starch is 0.1% by mass or more, preferably 0.15% by mass or more, and more preferably 0.2% by mass or more in terms of the pure polymer based on the total amount of the virus inactivating agent. If the content is less than 0.1% by mass, a sufficient virus inactivation effect cannot be obtained. The upper limit of the content of the cationic starch is not particularly limited, but typically 5% by mass or less, preferably 3% by mass or less, and more preferably 2% by mass or less.

In the present invention and the specification of the present application, "virus inactivation" refers to removing or significantly reducing the infectivity or the growth capacity of the virus. The virus that can be inactivated by the virus inactivating agent of the present invention is not particularly limited and various viruses can be inactivating targets regardless of the genome type or the presence or absence of an envelope.

Examples of the inactivating target include influenza virus types A, B, and C, isavirus, quaranjavirus, thogotovirus, rhinovirus, poliovirus, rotavirus, norovirus, enterovirus, hepatovirus, astrovirus, sapovirus, hepatitis E virus, parainfluenza virus, mumps virus, measles virus, human metapneumovirus, RS virus, Nipah virus, hendra virus, yellow fever virus, dengue virus, Japanese encephalitis virus, West Nile virus, hepatitis B and C viruses, Eastern and Western equine encephalitis viruses, rubella virus, Lassa virus, Junin virus, Machupo virus, Guanarito virus, Sabia virus, Crimean-Congo hemorrhagic fever virus, hantavirus, Sin Nombre virus, rabies virus, Ebola virus, Marburg virus, bat Lyssavirus, human T cell leukemia virus, human immunodeficiency virus, human coronavirus, SARS coronavirus, human parvovirus, polyomavirus, human papilloma virus, adenovirus, herpes virus, varicella zoster virus, EB virus, cytomegalovirus, smallpox virus, monkeypox virus, cowpox virus, molluscipoxvirus, and parapoxvirus; and the inactivating target is preferably enveloped single-stranded RNA viruses, more preferably influenza virus types A, B, and C, Isavirus, Quaranjavirus, and Thogotovirus that belong to family Orthomyxoviridae, and particularly preferably influenza virus.

Although the virus inactivating agent of the present invention exerts a sufficient effect by individually containing one or two or more essential oils selected from the group consisting of mint oil, eucalyptus oil, rosemary oil, sage oil, tea tree oil, getto oil, peppermint oil, lemongrass oil, cajeput oil, niaouli cineole oil, lime oil, lemon oil, lemon verbena oil, St. John's wort oil, ravintsara oil, rosewood oil, melissa/lemon balm oil, myrrh oil, mandarin oil, vetiver oil, frankincense oil, citronella oil, cardamon oil, and angelica oil, or cationic starch, the virus inactivating agent may comprise the essential oil and the cationic starch in combination as active ingredient.

The present invention provides a skin external-preparation composition comprising the above virus inactivating agent. The "skin external-preparation composition" of the present invention may be an external-preparation composition applied to the skin (including the scalp), and examples thereof include a cosmetic (including a base cosmetic and a makeup cosmetic), a cleanser, an external medicine, and an external quasi drug.

The skin external-preparation composition of the present invention may be in the form consisting of only the above-described virus inactivating agent, or in the form in which various components typically used in skin external-preparation compositions are appropriately contained as needed.

For example, the skin external-preparation composition of the present invention may comprise a lower alcohol (an alcohol having 6 carbon atoms or less) such as ethanol. It is known that a lower alcohol such as ethanol has inactivation effects on various viruses including influenza virus, and a virus inactivating agent composition with a high content of an alcohol is also known. However, in the skin external-preparation composition of the present invention, it is preferable that the content of an alcohol, if it is contained, be 50% by mass or less, preferably 40% by mass or less or 30% by mass or less, whereby people having sensitive skin can use it without anxiety.

Examples of other optional components that can be contained in the skin external-preparation composition of the present invention include an oil (such as animal and vegetable oils, mineral oil, ester oil, wax oil, silicone oil, higher alcohol, phospholipid, and fatty acid), a surfactant (anionic, cationic, amphoteric or nonionic surfactants), a vitamin (such as vitamin A group, vitamin B group, folate, nicotinic acid, pantothenic acid, biotin, vitamin C group, vitamin D group, vitamin E group, other ferulic acid, and γ-oryzanol), an ultraviolet absorber (such as p-aminobenzoic acid, anthranil, salicylic acid, coumalin, benzotriazole, tetrazole, imidazoline, pyrimidine, dioxane, furan, pyrone, camphor, nucleic acid, allantoin and their derivatives, amino acid-based compounds, shikonin, baicalin, baicalein, and berberine), an antioxidant (such as stearic acid ester, nordihydroguaiaretic acid, dibutylhydroxytoluene, butylhydroxyanisole, parahydroxyanisole, propyl gallate, sesamol, sesamolin, and gossypol), a thickener (hydroxyethyl cellulose, ethyl cellulose, carboxyethyl cellulose, methyl cellulose, carboxymethyl cellulose, sodium carboxymethyl cellulose, hydroxypropyl cellulose, nitrocellulose, polyvinyl alcohol, polyvinyl methyl ether, polyvinyl pyrrolidone, polyvinylmethacrylate, polyacrylate, carboxyvinyl polymer, gum arabic, tragacanth gum, agar, casein, dextrin, gelatin, pectine, and alginic acid and a salt thereof), a moisturizing agent (such as propylene glycol, 1,3-butylene glycol, polyethylene glycol, glycerin, 1,2-pentanediol, hexylene glycol, chondroitin sulfate and a salt thereof, hyaluronic acid and a salt thereof, and sodium lactate), a water-soluble polymer, a pH adjuster, an antiseptic/antifungal agent, a coloring agent, a cooling agent, a stabilizing agent, a microbial culture metabolic component, a blood flow enhancer, an antiphlogistic agent, an antiinflammatory agent, an anti-allergic agent, amino acid and a salt thereof, a keratolytic agent, an astringent, a wound healing agent, a deodorant, and various powder components. One of these components may be selected and added, or two or more thereof may be used in combination.

The dosage form of the skin external-preparation composition of the present invention may be any form and for example, may be in the form of a skin lotion, a cream, a salve, an emulsion, a foundation, an oil, a mask, a soap (including a medicated soap), a body soap, a lipstick, a fragrance, a facial wash, a deodorizer (such as underarm odor and foot odor), a bath agent, a shampoo, a conditioner, a hair tonic, and a hair spray.

The form of the skin external-preparation composition of the present invention may be a solution, a cream, a paste, a gel, a foam, a solid, or a powder, depending on the dosage form thereof.

The skin external-preparation composition of the present invention can be prepared in accordance with a usual method depending on the dosage form and the form, by comprising the virus inactivating agent mentioned above as an essential component.

### Examples

Hereinafter, the present invention will be described further in detail with reference to Examples, but the present invention is not limited by these Examples. The "%" in Examples refers to "% by mass" unless otherwise specified.

(Example 1)

### Inactivation Effect on Influenza Virus (No. 1)

### 1) Preparation of Samples (Test Solutions)

Samples (test solutions) of virus inactivating agents were prepared by the formulations shown in Table 1 below.

**[Table 1]**

| | Sample 1 | Sample 2 | Sample 3 | Sample 4 | Sample 5 | Sample 6 | Sample 7 | Sample 8 |
|---|---|---|---|---|---|---|---|---|
| Water | Balance | Balance | Balance | Balance | Balance | Balance | Balance | Balance |
| Citric acid (food products) | 0.02 | 0.02 | 0.02 | 0.02 | 0.02 | 0.02 | 0.02 | 0.02 |
| Sodium citrate | 0.08 | 0.08 | 0.08 | 0.08 | 0.08 | 0.08 | 0.08 | 0.08 |
| PEG-60 hydrogenated castor oil | 0.1 | 0.1 | 0.1 | 0.1 | 0.1 | 0.1 | 0.1 | 0.1 |
| 1,3-BG | 5 | 5 | 5 | 5 | 5 | 5 | 5 | 5 |
| Mint oil | - | 0.07 | 0.0233 | 0.007 | 0.03 | - | - | - |
| Rosemary oil | - | 0.032 | 0.0107 | 0.0032 | - | 0.03 | - | - |
| Eucalyptus oil | - | 0.03 | 0.01 | 0.003 | - | - | 0.03 | - |
| Sage oil | - | 0.02 | 0.0067 | 0.002 | - | - | - | 0.03 |
| Total | 100 | 100 | 100 | 100 | 100 | 100 | 100 | 100 |
| Amount of limonene | - | 0.0065 | 0.0022 | 0.0007 | 0.0013 | 0.0008 | 0.0016 | 0.0015 |

### 2) Test Method

Influenza virus type A (H1N1/PR/8/34) strain was used as a test strain.

To 1.080 mL of each sample (test solution) shown in Table 1, 0.12 mL (1 x 10⁴ pfu/mL) of the viral solution was added, and after reaction for 30 minutes, the solution was serially diluted 10-fold in SCDLP media and inoculated on canine kidney cells (MDCK) which were cultured in 96 well plates in advance. After culture under the conditions of 37°C and 5% CO₂, the number of plaques formed was measured to determine the residual virus infectious titer.

### 3) Test Results

The results are shown in Table 2. As shown in Table 2 below, in the combination of four mint-based essential oils (mint oil, rosemary oil, eucalyptus oil, and sage oil) (Sample 2), the logarithmic reduction value of the virus infectious titer was about 4 and it was shown to have a high inactivation effect on the influenza virus. On the contrary, in other samples (3 to 8) in which the amount of limonene was less than 0.006% by mass by reducing the number of essential oils or by reducing the amount of essential oils to be added, no virus inactivation effect was observed in any case.

**[Table 2]**

| | Virus infectious titer Logarithmic reduction value | Virus inactivation |
|---|---|---|
| Sample 1 | 0.459 | Poor |
| Sample 2 | 3.868 | Good |
| Sample 3 | 1.086 | Poor |
| Sample 4 | 0.818 | Poor |
| Sample 5 | 0.981 | Poor |
| Sample 6 | 0.937 | Poor |
| Sample 7 | 0.905 | Poor |
| Sample 8 | 0.823 | Poor |

### (Example 2)

Inactivation Effect on Influenza Virus (No. 2)

### 1) Preparation of Samples (Test Solutions)

Samples (test solutions) of virus inactivating agents were prepared by the formulation shown in Table 3 below.

**[Table 3]**

| | Sample 9 | Sample 10 | Sample 11 | Sample 12 |
|---|---|---|---|---|
| Water | Balance | Balance | Balance | Balance |
| Citric acid (food products) | 0.02 | 0.02 | 0.02 | 0.02 |
| Sodium citrate Na | 0.08 | 0.08 | 0.08 | 0.08 |
| PEG-60 hydrogenated castor oil | 0.1 | 0.1 | 0.1 | 0.1 |
| 1,3-Butylene glycol | 5 | 5 | 5 | 5 |
| Cationic starch (*1) | - | 0.24 | 0.024 | - |
| Cationic cellulose (*2) | - | - | - | 0.1 |
| Total | 100 | 100 | 100 | 100 |

| | | | | |
|---|---|---|---|---|
| (*1) Sensomer IC-50 (*2) Polymer JR-400 (manufactured by Union Carbide Corporation) | | | | |

### 2) Test Method

The test method was in accordance with the test method described in the above Example 1.

### 3) Test Results

The results are shown in Table 4. As shown in Table 4 below, in Sample 10 which contains 0.24% by mass of cationic starch (starch hydroxypropyltrimonium chloride) in terms of the pure polymer, the logarithmic reduction value of the virus infectious titer was 4 or more, and it was shown to have a significantly high influenza virus inactivation effect. On the contrary, no virus inactivation effect was observed in Sample 12 in which a similar amount (0.1% by mass) of a cationic cellulose derivative (hydroxyethylcellulose hydroxypropyltrimonium chloride) was contained in terms of the pure polymer, and in Sample 11 in which the content of the cationic starch was less than its effective amount (0.024% by mass).

**[Table 4]**

| | Virus infectious titer Logarithmic reduction value | Virus inactivation |
|---|---|---|
| Sample 9 | 0.459 | Poor |
| Sample 10 | 4.345 | Good |
| Sample 11 | 0.550 | Poor |
| Sample 12 | 0.720 | Poor |

Hereinafter, other formulation examples of the skin external-preparation composition according to the present invention will be listed.

### (Formulation Example 1) Skin external-preparation (virus block mist)

| Components included | % by mass |
|---|---|
| Ethanol | 20.0 |
| Cationic starch(*) | 1.0 |
| Citric acid | 0.02 |
| Sodium citrate | 0.08 |
| Water | balance |
| Total | 100 |

| | |
|---|---|
| (*) Sensomer CI-50 | |

An antivirus mist was obtained by discharging the skin external-preparation of the above-mentioned Formulation Example 1 by a mist dispenser.

Moreover, an aerosol product may be produced by using this formulation as a stock solution and by appropriately using a compressed gas (such as nitrogen, LPG, and carbonic acid).

### (Formulation Example 2) Skin external-preparation (gel)

| Components included | % by mass |
|---|---|
| Ethanol | 30.0 |
| Mint oil | 0.07 |
| Rosemary oil | 0.032 |
| Sage oil | 0.02 |
| Eucalyptus oil | 0.03 |

### (PEG-240/Decyltetradeceth-20/HDI)

| | |
|---|---|
| Copolymer | 0.5 |
| Carboxyvinyl polymer | 0.45 |
| 2-Amino-2-methyl-1,3-propanediol | 0.4 |

### (Acrylates/Alkyl Acrylate (C10-30))

| | |
|---|---|
| Crosspolymer | 0.05 |
| Polyquaternium-51 | 0.1 |
| Water | balance |
| Fragrance | 0.1 |
| Total | 100 |

(Formulation Example 3) Skin external-preparation (gel)

| | |
|---|---|
| Components included | % by mass |
| Hydroxypropylcellulose | 0.5 |
| Cationic starch (*) | 1.0 |
| Water | balance |
| Total | 100 |

| | |
|---|---|
| (*) Sensomer CI-50 | |

### (Formulation Example 4) Skin external-preparation (gel)

| Components included | % by mass |
|---|---|
| Ethanol | 10.0 |
| Mint oil | 0.07 |
| Rosemary oil | 0.032 |
| Sage oil | 0.02 |
| Eucalyptus oil | 0.03 |
| Carboxyvinyl polymer | 0.5 |
| Potassium hydrate | 0.2 |
| Glycerin | 10.0 |
| PEG-60 Hydrogenated Castor Oil | 0.1 |
| Water | balance |
| Total | 100 |

## Claims

1. A virus inactivating agent comprising, as active ingredient, one or two or more selected from the group consisting of mint oil, eucalyptus oil, rosemary oil, sage oil, tea tree oil, getto oil, peppermint oil, lemongrass oil, cajeput oil, niaouli cineole oil, lime oil, lemon oil, lemon verbena oil, St. John's wort oil, ravintsara oil, rosewood oil, melissa/lemon balm oil, myrrh oil, mandarin oil, vetiver oil, frankincense oil, citronella oil, cardamon oil, angelica oil, and cationic starch.

2. The virus inactivating agent according to claim 1, comprising, as active ingredient, one or two or more selected from the group consisting of mint oil, eucalyptus oil, rosemary oil, and sage oil.

3. The virus inactivating agent according to claim 2, comprising, as active ingredient, any three selected from the group consisting of mint oil, eucalyptus oil, rosemary oil, and sage oil.

4. The virus inactivating agent according to claim 2, comprising, as active ingredient, mint oil, eucalyptus oil, rosemary oil, and sage oil.

5. The virus inactivating agent according to any one of claims 1 to 4, wherein a total content of limonene is 0.006% by mass or more.

6. The virus inactivating agent according to claim 1, comprising the cationic starch as an active ingredient.

7. The virus inactivating agent according to claim 1 or 6, wherein the cationic starch is a cationic polymer represented by the following formula (I):
[Formula 1]
wherein X- represents an anion derived from an inorganic acid or an organic acid; a is 0.6 to 0.9 and b is 0.4 to 0.1, provided that a and b satisfy a + b = 1; and an average molecular weight is 30,000 to 1,000,000.

8. The virus inactivating agent according to any one of claims 1, 6, and 7, wherein an average molecular weight of the cationic starch is 100,000 to 500,000.

9. The virus inactivating agent according to any one of claims 1 and 6 to 8, wherein the cationic starch is starch hydroxypropyltrimonium chloride.

10. The virus inactivating agent according to any one of claims 1 and 6 to 9, wherein a content of the cationic starch is 0.1% by mass or more.

11. The virus inactivating agent according to any one of claims 1 to 10, wherein the virus is an enveloped single-stranded RNA virus.

12. The virus inactivating agent according to claim 11, wherein the enveloped single-stranded RNA virus is a virus belonging to family Orthomyxoviridae.

13. The virus inactivating agent according to claim 12, wherein the virus belonging to family Orthomyxoviridae is influenza virus.

14. A skin external-preparation composition comprising the virus inactivating agent according to any one of claims 1 to 13.

15. The skin external-preparation composition according to claim 14, further comprising an alcohol.

16. The skin external-preparation composition according to claim 15, wherein a content of the alcohol is 50% by mass or less.
